(19) Europäisches Patentamt
European Patent Office
Office européen des brevets

(11) **EP 2 498 676 B1**

(12) **EUROPEAN PATENT SPECIFICATION**

(45) Date of publication and mention
of the grant of the patent:
**15.07.2020 Bulletin 2020/29**

(51) Int Cl.:
*A61B 5/048* (2006.01)   *G06F 17/00* (2019.01)
*G06K 9/00* (2006.01)   *A61B 5/00* (2006.01)

(21) Application number: **10779177.4**

(22) Date of filing: **09.11.2010**

(86) International application number:
**PCT/US2010/055954**

(87) International publication number:
**WO 2011/059951 (19.05.2011 Gazette 2011/20)**

(54) **BRAIN ACTIVITY AS A MARKER OF DISEASE**

GEHIRNAKTIVITÄT ALS KRANKHEITSMARKER

ACTIVITÉ CÉRÉBRALE EN TANT QUE MARQUEUR DE MALADIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2009 US 615423**

(43) Date of publication of application:
**19.09.2012 Bulletin 2012/38**

(73) Proprietor: **Brainscope Company, Inc.
Bethesda, MD 20814-4481 (US)**

(72) Inventor: **CAUSEVIC, Elvir
Denver, CO 80209 (US)**

(74) Representative: **Finnegan Europe LLP
1 London Bridge
London SE1 9BG (GB)**

(56) References cited:
WO-A1-2008/148894   WO-A1-2009/045449
US-A1- 2008 208 073   US-A1- 2009 105 785
US-A1- 2009 220 429

**Description**

<u>Technical Field</u>

This invention relates to the field of disease monitoring, and more specifically, to systems using brain electrical activity as a marker of disease.

<u>Background</u>

Patients with a disease often display one or more specific symptoms. A symptom can provide an indication of the type of disease a patient is suffering from and tracking a symptom can provide an indication of disease progression. However, some diseases can be asymptomatic in some patients. Further non-specific symptoms may not clearly correlate with a specific type of disease or provide a useful indication disease progression. Brain diseases can be particularly difficult to monitor because they can be asymptomatic or the symptoms associated with them may be non-specific.

Brain diseases can have identifiable disease states, wherein a disease state may include a type, stage, or level of disease. Sometimes these disease states may correlate with time, such as, for example, ischemic stroke. Treatment timing for a stroke victim may be critical and some treatments, such as the application of tissue plasminogen activator, are commonly recommended only if applied within three hours of stroke onset. Thus, a "marker" of disease state associated with stroke can be time. However, such a traditional marker may be only statistically valid because disease progression can vary among patients. For example, a stroke victim who is a 30 year old non-smoking athlete will likely display a different disease progression compared to that of a 75 year old sedentary smoker.

Traditional disease state markers often rely on symptoms, time, biomarkers, imaging data, or other known indicators of disease state. A better indicator of disease state can be based on brain electrical activity. Such a marker of disease state could track, among others, discrete disease states or continuous levels of disease progression. For purposes of this application, a marker of a disease state based on brain electrical activity can be referred to, without limitation, a "neuromarker."

Biomarkers have been used as diagnostic indicators for many years, and have generally included substances that correlate with various diseases. For example, a biological substance, such as an enzyme, may be expressed in response to a disease. Other biomarkers may be inversely related to a disease, showing reduced levels when the disease is present. Yet other biomarkers may correlate with the progression of a pathological process, rising or falling as the disease progresses. Also, some biomarkers can be indicative of a physiological response to a therapeutic treatment. Thus, the appropriate time to apply and the effectiveness of pharmacological, electro-stimulatory, mechanical, or other treatments could be assessed using suitable biomarkers.

Traditionally, biomarkers have included biological molecules, such as, genes, proteins, or other biological factors. For example, apo-E4 has been linked to about 50% of patients with Alzheimer's disease. Imaging techniques can also been used as biomarkers. Computer tomography, magnetic resonance imaging (MRI), and positron emission tomography (PET) can be used alone, or in combination with other diagnostic data, to formulate suitable biomarkers for various diseases.

Recently, systems-based approaches utilizing multiple biomarkers have been developed. Diseases, such as cancer, are often multi-factorial. These diseases are often caused by interactions between multiple proteins, involve complex pathways, or are influenced by products of various genetic sources. To understand the nature of such disease mechanisms, genes have been analyzed using theories based on cooperative contribution to a phenotypic development. One example, using an information-theoretic definition of synergy, identified *ab initio* sets of interacting genes linked to a given phenotype (see Anastassiou (2007) "Computational Analysis of the Synergy Among Multiple Interacting Genes," Molecular Systems Biology, vol. 3:83). This definition of synergy, derived from a generalization of the concept of mutual information, connected two levels of organization (for example, genes and disease phenotype) to reveal the cooperative effects underlying a phenotypic state.

Another study applied network inference techniques to identify key pathways involved in prostate cancer progression (see Ergün, et al. (2007) "A Network Biology Approach to Prostate Cancer," Molecular Systems Biology, vol. 3:82). This study used a compendium of 1144 expression profiles spanning multiple cancer types to train a "mode-of-action by network identification" (MNI) algorithm. When tested on a set of prostate cancer profiles, an androgen receptor and several of its cognate target genes were identified as top genetic mediators.

Although computational analysis and sophisticated algorithms have been developed for biomarker assessment, the field is still limited to the molecular interactions of biomarkers. Because biomarkers must be extracted, isolated and quantified, any biomarker analysis is time-consuming and provides limited data. Further, a molecular response to a pathological condition may be delayed and may not correlate with the level of a disease. Thus, biomarkers often make poor prognostic indicators of a disease state. Biomarkers of brain disease may be particularly poor indicators because obtaining a suitable fluid sample can be difficult if it's cerebrospinal fluid, or the biomarker may interact with the blood-brain-barrier. Further, a specific biomarker of a brain disease can also be released by other biological processes. For example, S100, currently being investigated for TBI, can also be released after a bone is broken.

Similar to a biomarker's correlation with a disease state, a marker could also be associated with a brain state. Such a marker could find use as an indicator of neuronal pathology, or be used to track an appropriate time to apply a treatment regime, and subsequently track the effectiveness of treatment regimes, such as, for example, neuro-stimulation. Further, such a marker could be used to characterize emotions, cognitive ability, muscle reflexes, or other neuronal functions.

Determining suitable markers of brain state has been difficult given the complexity of brain functionality. Because brain function is predominantly electro-chemical in nature, rather than predominantly dependent on molecular interactions, biomolecular markers have limited neuronal application. Instead, neuro-imaging has been used extensively to study normal and diseased brain function.

Neuro-imaging has the advantage of being noninvasive, and large sets of imaging data, collected from numerous patient studies, have been analyzed using various mathematical techniques. For example, statistical methods applied to functional MRI data have been used to predict if a person lying (see Davatzikos, et al. (2005) "Classifying Spatial Patterns of Brain Activity With Machine Learning Methods: Application to Lie Detection," NeuroImage, vol. 28, p. 663-668). However, there are multiple disadvantages in using neuro-imaging. These include the low portability of imaging equipment, high equipment cost, and some forms of imaging expose the patient to unwanted levels of radiation.

Other studies have used electrical brain activity to monitor neuronal function during social interaction (see Tognoli, et al. (2007) "The Phi Complex as a Neuromarker of Human Social Coordination" PNAS, vol. 104(19), p. 8190-8195). Tognoli's behavioral study examined the interaction between two people using high-resolution spectral analysis of their brain activities, matched with relative measurements of the information the people exchanged. The study identified three distinct patterns of brain activity, one of which correlated with the presence or absence of social coordination.

While Tognoli's work may provide a useful indicator of social interaction under the controlled experimental conditions of their study, widespread application of their technique is limited. Their experiments used data gathered from sixty electrodes positioned around each participant's head, under precisely controlled, and uniform testing conditions. Also, various stimulatory cues provided control data specific for each participant, and data analysis of movement and behavioral events were precisely timed. Further, the study utilized high-resolution spectral data, gathered over long time periods and analyzed using spectral and time-frequency techniques that are computationally intensive.

Even though Tognoli's study has limited widespread application, a readily obtainable marker for normal or non-normal brain activity does have broad application. To be practical, the marker should require limited brain activity data gathered in short time. Further, the marker should be robust, obtainable during imprecise or uncontrolled conditions that arise in critical or everyday situations. Finally, the marker should be computationally efficient, permitting extraction or analysis by portable processors. Such a marker could see extensive application in emergency, military, sporting, hospital, or other environments.

Prior art Document WO 2009/045449 discloses a system and method of monitoring a mental state and analysing a brain signal to identify a mental state or condition using neural networks.

Accordingly, it is an object of the present disclosure to provide a neuromarker suitable for use with easy-to-use, self-contained, or portable systems for monitoring brain activity. Such systems and methods could find use in numerous applications, and provide real-time prognostic information to expert or non-expert users.

<u>Summary</u>

A first aspect of the present disclosure includes a method of monitoring brain activity. The method includes receiving a signal of brain electrical activity from a patient and processing the signal using at least one processor implementing an algorithm to extract a signal feature based on a brain state. The method also includes determining a neuromarker using at least one processor, wherein the neuromarker can be determined based on an association between the signal feature and a disease state.

A second aspect of the present disclosure includes a method of monitoring brain activity. The method includes receiving a signal of brain electrical activity from a patient. The method also includes processing the signal using at least one processor implementing a non-linear algorithm to extract a signal feature. A neuromarker may be determined using at least one processor based on an association between the signal feature and a library of features, wherein the library includes a plurality of signal features correlated with a plurality of disease states.

The invention relates to a system as defined in the appended claims.

A fourth aspect of the present disclosure includes a method of creating a library of features. The method includes receiving a signal associated with neuronal activity of a mammalian brain and processing the signal using at least one processor implementing a linear, a non-linear, or a combination algorithm to extract a signal feature. The method also includes associating the signal feature with a disease state, and storing the signal feature and the disease state in the library of features.

A fifth aspect of the present disclosure includes a method of assessing a neuromarker, wherein the method includes identifying a human brain with a known disease state, and receiving a signal associated with neuronal activity of the human brain. The method also includes processing the signal using a non-linear algorithm to extract a signal feature, correlating the signal feature with the known disease state, and identifying a neuromarker based on the signal feature.

It is to be understood that both the foregoing general description and the following detailed description are exemplary and explanatory only and are not restrictive. The accompanying drawings, which are incorporated in and constitute a part of this specification, illustrate several embodiments of the disclosed devices and methods.

Brief Description of the Drawings

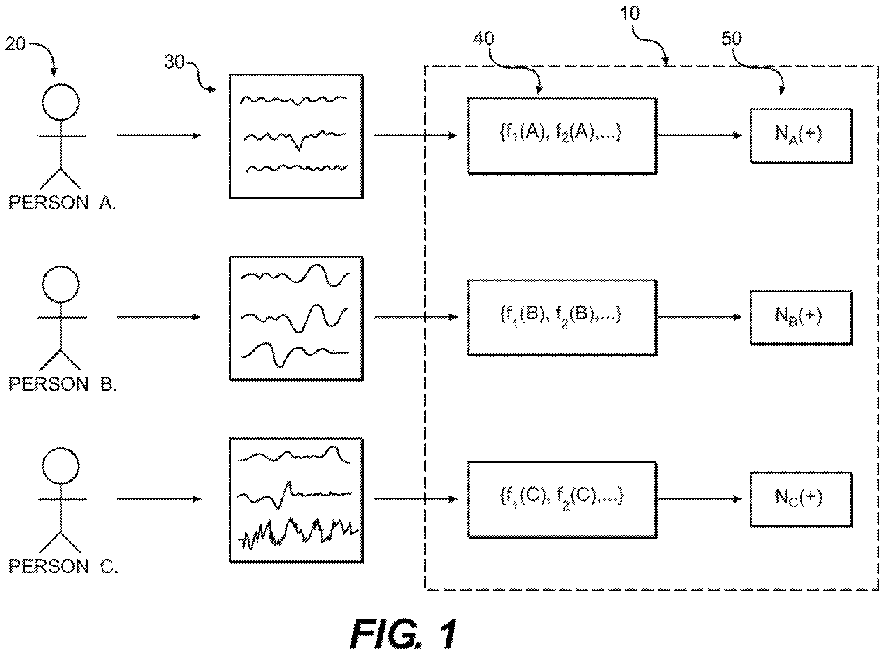

Fig. 1 illustrates a schematic diagram of a method for determining a neuromarker, according to an exemplary aspect of the present disclosure.

Detailed Description

Reference will be made in detail to the exemplary embodiments according to the present disclosure, examples of which are illustrated in the accompanying drawings. Wherever possible, the same reference numbers will be used throughout the drawings to refer to the same or like parts.

The present disclosure relates to systems and methods for providing a neuromarker, wherein the neuromarker may be used as a prognostic indicator of a disease state. The disease state, including non-normal physiological functioning, can be rapidly and accurately identified using systems and methods of the present disclosure. In particular, a disease state can be associated with one or more different stages of a disease, levels of disease, or one or more different types of disease. Also, each disease state may be associated with a particular brain state, as described in detail below. In some embodiments, such a brain state may include a plurality of brain electrical signals. Features of these signals may be extracted using non-linear methods, and a feature set may be associated with a neuromarker. Thus, the neuromarker may provide a time marker for a disease, based on brain-activity.

Figure 1 illustrates a system 10 configured to provide one or more neuromarkers, according to embodiments of the present invention. In particular, a number of patients 20 may provide various signals 30, wherein different signals are associated with the individual neuronal activity of each patient. As described in detail below, a set of features 40 may be extracted from one or more signals. A neuromarker 50 may be associated with each feature set 40, and may be used to provide a prognostic indicator of a disease state.

**Disease State**

In some embodiments, patient 20 may have one or more disease states. Generally, a disease state may include a type, stage, or level of one or more diseases, which can be discrete or continuous. A disease can include a pathological condition, a traumatic blow to the head, a bacterial, viral, or proteinaceous infection, or physiological mis-function. Also, a disease state may include a sub-type or certain stage of a disease. For example, cancer could be classified according to a "stage" of metastasis. Skin cancer can be classified as basal cell carcinoma and squamous carcinoma, and these types can be further classified into five separate stages from 0 - 4.

Disease states may also be classified by qualitative or quantitative levels. Specifically, a disease state may be considered chronic, acute, mild, or severe. Different levels of circulating antigens may also provide an indication of disease state, such as, for example, prostate-specific antigen.

A disease state could also include one or more diseases. For example, a patient with human immunodeficiency virus (HIV) may be co-infected with tuberculosis (TB). Each specific disease may also include a sub-type, stage, level, degree, or severity as outlined above. Thus, a disease state can represent a type of disease, a stage of a disease, a level of a disease, or a type, stage, or level for multiple diseases.

A disease state may also encompass other types of injury, such as, for example, a degenerative injury. For example, a degenerative brain injury can include Alzheimer's or Parkinson's disease. Other acquired brain injuries, such as, traumatic brain injury (TBI), including mild TBI, a hemorrhagic or ischemic stroke, tumor, hemorrhage, or encephalitis, can also be considered disease states. TBI can result from motor vehicle accidents, military incidents, sports injuries, or unwanted forces impacting the human head. Symptoms of a TBI may not appear until many days following an injury, and severe forms of TBI may require emergency treatment.

Disease state could also represent a recovery phase. For example, a recovery phase may include improved physiological function due to natural causes, drug treatment, electrical stimulation, mechanical assistance, or other type of therapeutic intervention. A mental disorder or mental condition may also be considered a disease state.

## Brain State

Generally, a brain state can be associated with brain activity, wherein brain activity can include an electro-physiological indicator of neuronal activity, such as a pattern of neural firing. Thus, specific temporal or spatial electrical activity of one or more collections of neurons can be represented by a brain state. For example, one or more brain electrical signals may be used to represent a brain state. Thus, the brain states of patients 20a, 20b, 20c can be represented by signals 30a, 30b, 30c, respectively. In addition, a brain state could be associated with a disease state, discrete or continuous.

In some embodiments, a brain state can include a representation of brain functionality. Specifically, a brain state can include an association between a pattern of neuronal electro-physiological activity and a physiological function. For example, a particular brain state could correlate with a cognitive function, such as memory, mental processing, or emotion. Brain functionality could also include neuronal activity associated with feedback or regulation of cardiac, gastric, endocrine, or other physiological processes. In particular, a brain state could be associated with muscle movement or sensory perception.

In other embodiments, a specific brain state could be associated with a particular disease. For example, a certain type of dementia could have a specific brain state. Various neuro-pathologies could have particular brain states, including epilepsy, multiple-sclerosis, Lou Gehrig's disease, or Huntington's disease. Also, other pathologies, such as cancer, heart disease, or pulmonary disease could have distinct brain states.

A correlation between a brain state and a disease state can be readily discernible for neuronal disorders. For example, as described below in more detail, analysis of certain frequency bands of brain signals can be associated with Alzheimer's disease. It can be more challenging to associate a brain state with a disease state when the disease is non-neuronal, such as, for example, HIV, TB, cancer, or the common flu. The present disclosure provides a technique to associate neuronal and non-neuronal disease states with discrete brain states based on brain activity signals. Based on this association, a prognostic indicator of a disease state can be determined. In other embodiments, an appropriate time to administer treatment and then to track the efficacy of that treatment can be determined.

## Brain Electrical Signals

The human brain is a highly complex organ, including numerous interconnected neuronal cells. The brain operates to enable conscious behavior, thought, and emotion. The brain is also connected to the peripheral nervous system, nerve cells that extend throughout the human body. Both the peripheral and central nervous systems operate in concert to regulate muscular movement and control physiological functionality.

Neuronal cells are capable of forming interconnections with adjacent neurons via dendritic processes and synaptic terminals. Neuronal cells communicate via action potentials, electro-chemical currents that propagate via the select movement of ions across cellular membranes. Synaptic terminals release various factors that function to transmit the signal from one neuron to the next. Part of this activity can be conducted to the surface of the cortex, where it can be recorded as an electroencephalogram (EEG) signal. Other types of signals could be also obtained and used with the systems and methods described herein, including a magneto-encephalography (MEG) signal. Signals of brain activity can contain physiological or pathological information pertaining a patient's disease state.

A brain's electrical activity represents underlying neuro-physiological activity. Signal of such activity can be obtained from a set of electrodes placed on the surface of a person's head, such as described by U.S. Patent Application Serial No. 12/059,014. The predominant component of a brain electrical signal is generated by excitatory postsynaptic potentials, though action potentials of cortical neurons also contribute to the signal. Signal rhythms generally result from excitatory and inhibitory postsynaptic potentials produced in the cerebral cortex.

A brain electrical signal is mathematically complex, in part because the signal represents the combination of signals originated from numerous individual neurons. A brain electrical signal can be generally described as noisy and pseudo-stochastic. The signal is generally between about 10 to about 300 $\mu$V in amplitude, making it prone to contamination from other physiological or electrical noise. Some brain signals evoked by audio stimulation, such as auditory brainstem response, can have meaningful amplitudes in the tens of nanovolts. Also, artifacts from cardiac, ophthalmic, muscular, or recording systems can further contaminate a brain electrical signal.

Brain electrical signals often display high degrees of randomness and non-stationarity, whereby the signal varies with physiological state. For example, in certain pathologies, such as during epileptic seizures, a signal may show non-stationarity features or singularities. Further, brain signals can be highly non-linear. Although traditional linear techniques can be applied to analyze brain electrical signals, they are generally non-linear in time, state, and site of origin within the brain.

Some components of a brain electrical signal are transient rather than rhythmic. Spikes and sharp waves may represent seizure activity or interictal activity in individuals with epilepsy or a predisposition toward epilepsy. Other transient components are associated with normal brain function, including vertex waves and sleep spindles which are seen in normal sleep.

Some types of brain activity which are statistically uncommon but are not associated with dysfunction or disease. These are referred to as "normal variants," and includes the "mu rhythm." Also, a normal brain electrical signal can show variation with age. For example, a neonatal brain electrical signal can be distinct from an adult brain electrical signal, and a childhood brain electrical signal generally includes slower frequency oscillations than an adult signal.

A brain electrical signal can be described in terms of rhythmic activity, that can be further divided into bands by frequency. These frequency bands are termed Delta, Theta, Alpha, Beta, and Gamma. These designations arose because rhythmic activity within a certain frequency range was noted to have a certain distribution over the scalp or a certain biological significance. For example, the cerebral component of a brain electrical signal falls generally in the range of about 1 to about 20 Hz, in part because activity below or above this range can be artifactual under most standard clinical monitoring conditions. High frequency brain activity may also correlate with states of consciousness that have been clinically examined and shown to be of general diagnostic value. These frequencies would include frequencies beyond about 100Hz.

The Delta band includes the frequency range up to about 3 Hz, and is generally the highest in amplitude and lowest in frequency. Delta bands are normally observed in adults in slow wave sleep or in babies. They may occur focally with sub-cortical lesions and in general distribution with diffuse lesions, metabolic encephalopathy hydrocephalus, or deep midline lesions.

The Theta band includes the frequency range from about 4 Hz to about 7 Hz. Theta is normally associated with young children, and may be correlated with drowsiness or arousal in older children and adults. Excess Theta levels for a specific age can represent abnormal activity. Also, Theta bands can manifest as a focal disturbance in focal sub-cortical lesions, and in generalized distribution in diffuse disorders, metabolic encephalopathy, deep midline disorders, or some instances of hydrocephalus.

The Alpha band includes the frequency range from about 8 Hz to about 12 Hz. This activity is seen in the posterior regions of the head on both sides, being higher in amplitude on the dominant side. The Alpha band can also be associated with eye closure and relaxation, and attenuates with eye opening or mental exertion. Alpha can also be indicative of abnormal functionality. For example, a brain electrical signal with diffuse Alpha occurring in coma and non responsive to external stimuli is referred to as an "Alpha coma."

The Beta band includes the frequency range from about 12 Hz to about 30 Hz. Beta is usually localized on both sides of the brain in symmetrical distribution and is predominately frontally. Low amplitude Beta with multiple and varying frequencies is often associated with active, busy, or anxious thinking, and active concentration. Rhythmic Beta with a dominant set of frequencies is associated with various pathologies and drug effects, especially benzodiazepines. Beta may be absent or reduced in areas of cortical damage, and is generally the dominant rhythm in alert patients.

The Gamma band includes the frequency range about 26 to about 100 Hz.. Gamma rhythms are generally associated with different populations of neurons functioning as networks to conduct certain cognitive or motor functions.

High frequencies beyond about 100Hz have been correlated with states of consciousness. These include changes of consciousness as a result of anesthesia administration. Clinical evidence has indicated that the high frequency regions of an EEG signal may be indicative of other pathologies or changes in various disease states.

**Signal Processing**

While brain electrical signals can be analyzed as discrete frequency bands, processing of brain electrical signals can be complicated because they are often composed of multiple oscillations, often non-linear and non-stationary in nature. These oscillations can have different characteristic frequencies, spatial distributions, or associations with different states of brain functioning (such as awake vs. asleep). Brain electrical signals are often further complicated by sources of noise or artifacts. These sources of noise and artifacts can readily affect brain electrical signals in part because neuronal activity usually occurs at about 10 to about 100 $\mu$V, similar to the sources of interference.

Brain electrical signal interference can arise from a number of different sources, depending upon the environment of the patient or the patient's state. Interference can originate from power lines (50/60 Hz), TV stations, radio, wireless phone, or other sources. Other interference can also arise from the patient. These can include body movements, such as, for example, blinking, jaw movement, tongue, or head movement. Muscle artifacts and heart beats can also provide interference.

Some signal interferences are easier to remove than others. For example, external sources can typically be removed with suitable use of notch filters, or by properly grounding or shielding the monitoring system. Other filters can operate to remove some physiological interferences, as known in the art. However, other sources of interference, such as, for example, rapid eye movement and electrocardiogram (ECG) signals, can be more difficult to reject. Eye and ECG artifacts can overlap in amplitude and spectrum of brain electrical signals, and sometimes can interfere with signal analysis.

Normal brain electrical signals are typically about 1 to about 1.5 Hz oscillations, and second-order harmonics are about 2 to about 3 Hz, placing them in the Delta band. In some pathologies, such as ischemia, the brain electrical signal may be weak, and the influence of the ECG signal on the brain electrical signal can be significant. Also, brain injury following cardiac arrest can show ECG artifacts during the ischemia period and early recovery phase.

**Signal Pre-processing**

Prior to signal processing, one or more pre-processing steps may be applied to the brain electrical signal. For example, a brain electrical signal may require denoising, filtering, windowing, sampling, or digitizing. In particular, artifact identification and removal may use a signal processing method as described in commonly-assigned U.S. Patent Application No. 12/106,699. Artifact identification and rejection can require transforming a signal into one or more components, computing their fractal dimension, identifying noise components based on their fractal dimension, attenuating the identified noise components, or reconstructing a denoised signal using inverse transform.

Initially, a brain electrical signal can be digitized and then deconstructed into constitutive coefficients using a linear or non-linear transformation method, such as, a Fast Fourier Transform (FFT), an Independent Component Analysis (ICA) transform, a wavelet transform, or a wavelet packet transform. Suitable methods are described in commonly assigned U.S. Patent Application Serial No. 11/195,001 titled "Method For Assessing Brain Function and Portable Automatic Brain Function Assessment Apparatus," U.S. Patent Application Serial No. 12/041,106 titled "Field-Deployable Concussion Detector," and U.S. Patent Application Serial No. 12/106,657 titled "System and Method For Signal Denoising Using Independent Component Analysis and Fractal Dimension Estimation,". The fractal dimensions of the coefficients can then be calculated in the transform domain, and the coefficients that have fractal dimensions higher than a threshold value attenuated. The intact and re-scaled coefficients can then be remixed using an inverse transform to generate a denoised signal. Such a signal can then be further processed to extract features and classify the extracted features, as described in detail below.

In some embodiments, a wavelet transformation can be used to perform an signal denoising operation prior to a feature extraction. Optional denoising can use wavelet coefficient thresholding to separate incoherent noise from the coherent signals. Specifically, a wavelet transform can be performed on a brain electrical signal to obtain a number of wavelet coefficients at different scales. Threshold levels can be set for various noise components, and any coefficient below these thresholds can be set to zero or reduced. As such, wavelet transformation of brain electrical signals can provide fast and efficient denoising for rapid feedback while monitoring a patient's brain activity. Wavelet transformations do not generally require heavy computational demands, or large amounts of computer memory, and can facilitate application in small, portable devices.

In operation, the wavelet transform can include an integral transform that projects the original brain electrical signal onto a set of unconditional basis functions called wavelets. The transformation can use a discrete waveform, an orthogonal wavelet, a bi-orthogonal wavelet, or some wavelets may be continuous. Also, the wavelet transform can be used to obtain a number of wavelet coefficients at different scales. In some embodiments, a series of different wavelets may be used for denoising, feature extraction, or other signal processing.

Many types of wavelets which may be used to develop a wavelet transform, and various types of wavelet transforms exist. Various other de-noising algorithms and data removal techniques may also be employed. For example, suitable de-noising techniques are described in U.S. Patent Nos. 7,054,453, 7,054,454, 7,302,064, 7,333,619, and International Publication No. WO 2006/034024.

**Signal Feature**

As previously described, a signal can include a measure of neuronal activity associated with a mammalian brain. For example, as shown in Fig. 1, signals 30a, 30b, 30c are associated with the brain activity of patients 20a, 20b, 20c, respectively, and can represent a brain state of each patient. Further, signals 30 can provide a source of patient-specific data from which one or more features may be extracted. For example, features $f_1(A)$ and $f_2(A)$ are associated with patient A, $f_1(B)$ and $f_2(B)$ are features specific to patient B, and features $f_1(C)$ and $f_2(C)$ are specific to patient C. One or more features associated with patients 20a, 20b, 20c can be described by feature set 40a, 40b, 40c, respectively. In some embodiments, a single feature or a feature set 40 can be used to determine one or more neuromarkers.

A signal feature can include any readily identifiable component, or processed component, associated with a signal representative of neuronal activity. For example, a feature could include an amplitude, frequency, period, phase, real or imaginary component of a brain electrical signal recorded from the skull of a patient. Additionally, a signal feature could include a statistical parameter associated with a signal associated with brain activity, such as, for example, an average, mean, standard deviation, or other statistical measure of one or more signals. Other statistical methods can include t-test, chi-square, ANOVA, regression analysis, factor analysis, and time series analysis. In some instances, a feature can include a quantifiable measure of a signal associated with brain activity. Any signal feature, or representation of a feature, could be stored in a database for later use, as described in detail below.

In some embodiments, a feature could be derived from a brain electrical signal. For example, a signal feature could be derived by integrating, differentiating, or applying a mathematical function to a brain electrical signal. Such processing can be used to determine an area under a brain electrical signal, a gradient of a brain electrical waveform, or other parameter associated with the brain electrical signal. For example, a Fourier transform (FT) could be applied to a brain electrical signal. Based on FT processing, a feature could include a real or complex number, time, frequency, vector, matrix, harmonic, z-score, eigenvalue, or other parameter derived from FT processing.

In other embodiments, a signal feature could include a parameter derived from the application of one or more algorithms. For example, a feature could include a variable associated with linear or non-linear processing of a brain electrical signal. In particular, a feature could be derived from the application of wavelet, wavelet-packet, diffusion wavelet, or fractal mathematics techniques. Also, a signal feature could include a waveform, cloud, cluster, or other representation associated with non-linear processing of a brain electrical signal. In addition, a signal feature could further be associated with a partition of data, subset of data, or combination of multiple data.

**Signal Feature Extraction**

In some embodiments, a feature can be extracted from a brain signal, before, after, or during a processing step. For example, a signal feature could include a variable associated with an unprocessed signal, obtained before a brain electrical signal is processed. Such "raw" features could be extracted from Delta, Theta, Alpha, Beta, Gamma, or high frequency bands. Signal features could also be extracted via a processing step. For example, data from a brain electrical signal could be removed by a processing step, and the removed data could be used, or further processed, as a feature. Also, filtered, sub-threshold, noise, or other data could be used to extract a feature.

In certain instances, a feature could also be extracted following brain electrical signal processing. As previously described, various filters, algorithms, or other data processing techniques can be applied to a brain electrical signal. Following, various processed data are available for further analysis. Such processed data may also be used to determine a signal feature as described above for a feature associated with an unprocessed brain electrical signal. For example, a feature could include the amplitude of a waveform created by processing a brain electrical signal using a wavelet analysis technique. Another signal feature could be based on spectral analysis of such a waveform, or additional processing of a previously processed signal.

A signal feature can be analyzed using various mathematical methods. For example, multiple signal features could be subject to statistical measures to determine average, standard deviation, and other statistical measures, as outlined above. The signal feature could be derived from a single brain electrical signal or a combination of brain electrical signals. Further, a spatial collection or time series of features could be analyzed. For example, a feature could be obtained from a brain electrical signal obtained from only the left hemisphere of the brain, only the right hemisphere, or from two signals from both hemispheres. A feature could also be extracted from brain electrical signals obtained at different times. For example, brain electrical signals obtained before and after a stimulus has been applied to a patient may be used to determine a feature.

In some instances, a signal feature can be extracted following data removal from a brain electrical signal, while in other instances a "raw" brain electrical signal can used. As described in more detail below, linear or non-linear signal processing techniques can be used to extract a feature. Such techniques can include, for example, the use of wavelet-packets, diffusion wavelet processing, or fractal mathematics. For example, suitable wavelet-packet techniques are well known. In addition, suitable diffusion wavelet techniques are described in commonly-assigned U.S. Patent Application Serial No. 12/105,439 titled "Method and Apparatus for Assessing Brain Function Using Diffusion Geometric Analysis." Suitable fractal mathematics techniques are described in commonly-assigned U.S. Patent Application Serial Nos. 12/106,699 and 12/106,657, titled respectively "System and Method for Signal Processing Using Fractal Dimension Analysis" and "System and Method for Signal Denoising Using Independent Component Analysis and Fractal Dimension Estimation." In addition, other advanced processing techniques may be employed, as described, for example, in commonly-assigned U.S. Patent Application Publication No. 2007/0032737A1.

In some embodiments, brain electrical signal processing can include extracting one or more features from a denoised brain electrical signal. For example, a feature extraction algorithm can be configured to perform a linear feature extraction algorithm based on FFT and power spectral analysis, according to a method disclosed in commonly-assigned U.S. Patent Application Publication No. 2007/032737, and U.S. Patent Application Serial No. 12/041,106.

A linear algorithm could be configured to extract a feature by Fourier transforming a frequency band and calculating the power of the frequency band. The frequency composition can be analyzed by dividing the signal into Delta, Theta, Alpha, Beta, or Gamma bands as previously described. In some instances, higher frequencies up to and beyond 1000 Hz may also be used. A univariate signal feature can then be determined by calculating the absolute and relative power for each electrode or between a pair of electrodes within a select frequency band. Following, an asymmetry and coherence relationship among the spectral measurements can be determined. In some instances, multivariate features derived from non-linear functions of univariate features may also be used. Such measures can be age-regression normalized, or Z-transformed to extract features (Z-scores) for discriminant analysis.

In another embodiment, not being part of the invention, a linear feature extraction algorithm can be based on wavelet transforms, such as Discrete Wavelet Transform (DWT), Continuous wavelet transform, or Complex Wavelet Transforms (CWT). Although Fourier analysis often provides a less computationally demanding method of signal processing and feature selection, transitory information can be lost in the frequency domain. FFT-based spectral estimation assumes a stationary and slowly varying signal, however brain electrical signals can be time-varying, transient (e.g. spikes/bursts), or non-stationary. Fourier transforms can provide rhythmic frequency information, but may not reveal temporal frequency data. If time localization of a spectral component is required, a transform should provide a time-frequency information. Wavelet analyses are well-suited for such application because of their high time-frequency resolution and low computational complexity.

According to the invention, signal feature extraction uses a non-linear signal transform method, such as a wavelet packet transform. Such a transform can extract a Local Discriminant Basis (LDB) feature, wherein a LDB algorithm can define a set of features that are optimized for statistical discrimination between different classes of signals. These signal features are initially calculated using power spectral densities over a set of epochs associated with each electrode channel. For each patient, the algorithm produces one power spectrum per channel, and then power spectra quotients for each pair of channels are calculated. For example, a five channel system produces fifteen power spectra per subject, permitting calculation of fifteen distinct bases, or sets of LDB vectors. An LDB feature can then be determined using a wavelet packet table for each power spectrum and a Haar or other standard or custom wavelet function. The function can be applied to low and high pass sub-bands, generating a tree structure of possible wavelet packet bases. Accordingly, signals can then be decomposed into a time-frequency dictionary.

In another embodiment consistent with the present disclosure, diffusion geometric analysis can be used to extract a non-linear feature according to a method disclosed in commonly-assigned U.S. Patent Application No. 12/105,439. Initially, brain electrical data set can be organized into a plurality of digital documents, each document including a time window of temporal information associated with each electrode. Affinity between the documents may then be computed using an appropriate affinity matrix $A$. The affinity matrix $A$, between a document at time $i$ and a document at time $j$ may be defined as:

$$A_{i,j} = \frac{e^{\frac{-\|v(i)-v(j)\|^2}{\varepsilon}}}{w(i)w(j)}$$

wherein $\varepsilon$ is a threshold parameter, $w(i)$ is a weighting function at time $i$, $w(j)$ is the weighting function at time $j$, and the weighting functions are selected such that $A$ is Markov in $i$ and $j$. Next, the eigenvectors of the affinity matrix can be determined and used to construct a Euclidean space representing the diffusion geometry of the dataset including a plurality of diffusion coordinates. If the first three eigenvectors are used, an embedding in three dimensional Euclidean space can be obtained wherein the diffusion metric, or relational inference, can be isometrically converted to a corresponding Euclidean distance. A feature may be obtained based on the metrics provided by the diffusion geometry analysis.

**Feature Library**

A feature may be determined based on various criteria. For example, a predetermined portion of the diffusion coordinates space may be partitioned into data corresponding to a particular feature. In another embodiment, applying diffusion geometric analysis to multiple digital documents may result in a formation in multidimensional space, such as, for example, a cluster. The cluster could be initialized based on one metric, and then hierarchically aggregated based on a different metric from the multiplicity of metrics corresponding to the diffusion distances. Such a cluster may represent a specific feature, part of a feature, or set of features, depending on the metrics used to initialize the cluster.

Each set of features can include one or more features extracted as described herein. Thus, feature sets 40 can provide a representation of various brain states, wherein feature set 40a, 40b, 40c can be associated with a brain state of patient 20a, 20b, 20c, respectively. Different brain states can further be associated with various neuronal and non-neuronal disease states. Therefore, a neuromarker determined using a feature set can be correlated with a specific disease state.

In some embodiments, feature sets 40 can be selected from a features library, wherein the library of features can include a plurality of features. The plurality of features can be associated with various disease states such that one or more features can be associated with a specific disease state.

An association between a signal feature and brain state can include a statistical association, a correlation, a comparison, or similar relationship. For example, one or more features could be associated with a disease state by gathering signal data for many patients with a known disease. The patient population data may be processed using the non-linear methods described herein. Statistical analysis of this processed data could then be used to identify one or more features that indicate a particular disease state. In other instances, correlative techniques could be used wherein the features of two or more disease states are correlated. Such a correlation may permit prognostic evaluation of a patient without having obtained features specific for the patient's particular disease state. Feature comparison could also be used to determine an association. For example, a feature could be associated with blood pressure and a certain disease could be known to affect blood pressure. Tracking the blood pressure feature could then provide a comparable indication of the progression of the disease.

To create a library of features, a signal associated with neuronal activity of a mammalian brain may be received using electrodes described herein. The patient may have a known disease state or be undergoing a disease treatment. Non-linear processing of the signal is used to extract a signal feature. Following, the signal feature may be associated with the patient's disease state. Lastly, the signal feature and the disease state may be stored in a library of features, as further described below.

A feature set can be derived using any suitable algorithm, such as, for example, a genetic algorithm. Genetic algorithms are a form of evolutionary algorithm based on concepts of evolutionary biology, including inheritance, mutation, selection, and crossover. In application, genetic algorithms can be used to find exact or approximate solutions. Such algorithms are described in commonly-assigned U.S. Patent Application No. 12/541,272 titled "Development of Fully-Automated Classifier Builders for Neurodiagnostic Applications,".

**Neuromarker Determination**

Neuromarkers based on brain activity offer several advantages over traditional biomarkers. Neuromarkers can provide a rapid indication of brain state as signals associated with neuronal activity can be quickly obtained and analyzed. Such real-time, or near real-time, analysis avoids delays due to chemical analysis or biological assays. In addition, more information can be gleaned from signal data due to their complex nature, while biomarkers are limited to quantitative or qualitative evaluation. Given suitable processing of brain signal data, a subsequently derived neuromarker could find use as a prognostic indicator of neuronal or non-neuronal disease states.

In some embodiments, a neuromarker 50 could be associated with one or more signal features. For example, a signal feature "f" could be extracted using diffusion geometry, Local Discriminant Basis, or other method as previously described. Such a feature, or set of features 40, may be associated with a neuromaker correlated with a specific disease state. In particular, neuromarkers $N_A$, $N_B$, $N_C$ may be associated with a disease state correlated to feature sets 40a, 40b, 40c, respectively. Thus a time marker for brain activity, $N_A(t)$, $N_B(t)$, $N_C(t)$ can be associated with Patients A, B, C, respectively. For example, a feature set extracted from spectral analysis of Delta, Theta, Alpha and Beta bands could be associated with a neuromarker for Alzheimer's disease. According to the invention, various signal features are extracted from brain electrical signals using non-linear algorithms.

According to the invention, neuromarker 50 functions as a disease indicator, wherein the neuromarker provides an indication of presence or absence of a disease state. Such a classification is based on Linear Discriminant Analysis (LDA). LDA can combine one or more Z-scores into a discriminant score. Prior to application of LDA, one or more extracted features are age-regressed and z-transformed. According to the invention, LDA employs a two category classifier to assign a discriminant score between 1 and 100 to each subject. A score of less than 50, for example, can indicate that the subject is more likely to exhibit a certain disease state than not exhibit that disease state. Other scores could be associated with one or more brain states. Yet other examples include classifications of normal or abnormal physiological function, mild or severe disease state, acute or chronic condition, and so forth.

According to the invention, discriminant scores, $S_A$ and $S_B$ corresponding to different states $A$ and $B$, are computed for any subject with the following Fisher LDA formulas:

$$S_A = \frac{100.G(1)}{(G(1) + G(2))}, S_B = \frac{100.G(2)}{(G(1) + G(2))}$$

$$G(1) = e^{(Z.W_A + C_A)}, G(2) = e^{(Z.W_B + C_B)}$$

wherein $Z$ denotes a set of age-regressed and z-transformed features computed for a patient. $W_A$ and $W_B$ denote two weight vectors derived from a reference database, and $C_A$ and $C_B$ are bias (threshold) constants. The reference database can include a "BrainBase Database" according to the Bx™ technology.

In some embodiment, the weights can be pre-selected using a training routine to provide appropriate separation between the states, as well known in the art. Weights for univariate or multivariate features may be estimated from a population reference database. Such a database can include normative data indicative of brain activity associated with a disease state. Also, such a database could include data associated with general patient information, medical history, or prior treatment. Data could also include objective or subjective patient data associated with their brain activity, or with various types or severities of diseases. In particular, the weights may be selected from a database of the subject's brain activity data generated in the absence or presence of a specific disease state.

The discriminant scores can be further converted to probabilities of a correct or incorrect classification using Receiver Operating Characteristics (ROC) curves. The ROC curves can indicate the threshold, sensitivity, specificity, positive predictive value (probability of the disease when the classification result is positive), or negative predictive value (probability of no disease when the classification result is negative) that can be expected from a particular algorithm or classifier.

An ROC curve can be described as a curve through a set of points: {(1-specificity(T), sensitivity(T))}, which can be obtained by varying a critical value, or threshold (T), between 0 and 1. Thus, ROC curves can illustrate an achievable statistical performance of a classifier, dependent on the selected critical value. Other types of classifier may also be used, including a Partial Least Squares or quadratic classifier.

In some embodiments, neuromarker 50 could be used as a prognostic indicator. Specifically, neuromarker 50 may be used to monitor the progression of a disease by tracking one or more features associated with a brain state. Also, such a neuromarker could be used during drug or other therapies to provide an index to monitor disease progression or modify treatment.

For example, a discriminant score as described above could be used to provide a quantitative neuromarker, wherein the level of neuromarker 50 could be measured on a scale. Such a scale can then be used to generate an index associated with the neuromarker. The neuromarker index could also be calibrated to correspond to a level of a disease, and thus used to track disease progression. For example, a disease level ranging from absent, to slight, mild, moderate, or severe could be established. This index may be used in combination with disease states associated with a patient's brain activity. Thus, a neuromarker index may be used to track disease progression, wherein increasing or decreasing values of the index may reflect the improving or worsening of a disease state. This information could be displayed, stored, transmitted, or used to modify a treatment regime.

A prognostic neuromarker could be based on one or more features, as described above. Disease progression could also be associated with the creation or destruction of one or more features 40. For example, a new feature may arise during disease progression. In some instances, a feature detected in a normal patient may reduce or cease during disease progression. Such features could also shift temporally, spatially, or in other ways.

In some instances, neuromarker 50 could be associated with statistical analysis. For example, the average, or standard deviation of a feature or set of features 40 may change with the progression of a disease state. Also, prognostic indicators could be obtained by application of one or more algorithms, as previously described. For example, one or more features associated with non-linear feature extraction may be further processed using various algorithms to provide a suitable neuromarker.

In other embodiments, neuromarker 50 could be based on a coherence value, wherein the coherence value is associated with variation of one or more features. Coherence generally describes a correlative relationship between a property of one or more features. For example, coherence can include temporal or spatial properties of a feature. Temporal coherence can include a correlative measure between the value of a feature at two different times, while spatial coherence can describe a cross-correlation between geometric properties.

In some embodiments, a coherence value may be associated with one or more time points. For example, a first coherence value may be determined at a first time $t_1$. Further, a second coherence value may be determined at a second time $t_2$. If the coherence values display divergent behavior over time, the comparison between feature of interest and relevant data set may be considered less probable. By comparison, if the coherence values display convergence overtime, the comparison between the feature and data may become more probable, increasing the likelihood that the feature of interest is associated with the corresponding brain state. Such temporal analysis can be interpolated or extrapolated to provide additional correlative data.

These and other techniques may be employed to further refine the determination of neuromarker 50. In some instances, one or more features may increase or decrease in value, frequency, amplitude, morphology. Other features may change relative to yet other features. For example, degrees of dispersion, clustering, or cloud form may change. Such change could occur over time, spatial origin, or in another manner associated with one or more features.

In some embodiments, one or more neuromarkers may be correlated with other data. For example, a neuromarker may be correlated with a traditional disease state marker, such as, a biomarker, time, or imaging data. Such a correlation may use any suitable algorithm, such as, for example, an algorithm based on mutual information. In information theory, the mutual information of two random variables can include a quantity that measures the mutual dependence of the two variables. For two discrete random variables X and Y, mutual information can provide a measure of the information that X and Y share. As such, knowing one of these variables can reduce the uncertainty about the other variable.

**Neuromarker Storage**

Signals associated with brain activity typically contains large amounts of information. Multiple electrodes positioned about the skull provide spatially independent sources of information, and considerable data accrues as signals are collected over time. Such large amounts of information can quickly overwhelm existing data storage devices.

Large storage systems are usually cumbersome and power intensive, reducing their portability. Thus, storage of a large amount of raw brain signals is usually not practical for portable monitoring systems. Because a feature set, or collection of feature sets, require less storage space than is required for storage of raw brain signals, the present invention can facilitate portable brain monitoring. Rather than storing and analyzing brain electrical signals, the present method can utilize signal features derived from brain electrical signals, reducing the storage and processing requirements of the monitoring devices.

The reduced data size required for feature-based monitoring permits accurate and precise brain activity assessment. For example, a larger number of features, or additional data for each feature, could be obtained and stored. Extraction and analysis of these features could permit an accurate assessment of a patient's brain state. Some features can include additional spatial, temporal, or different types of features associated with brain signals.

Data representative of one or more features, neuromarkers, disease states or brain states may be stored in any suitable data format, such as, for example, a relational database. These or other parameters may be inter-related or associated as required. In some instances, the association of these parameters can include a statistical association, a correlation, or a comparison.

In some embodiments, a database may be configured to store multiple neuromarkers, or representations of neuromarkers. Neuromarker representations can include statistical measures, time series, or other data formats. Storage of neuromarkers may be required for tracking or predicting a disease state. Also, neuromarker data could be transmitted to remote locations for prognostic evaluation. Further, additional diagnostic information about the patient, disease, disease state, symptom, diagnostic measures, or other information relevant to the patient can be stored together with the neuromarker information, and possibly be used in conjunction with the neuromarker.

**Neuromarker Application**

Neuromarker 50 can be used in a range of medical applications. For example, neuromarker 50 could be displayed to provide a care giver with prognostic information, wherein the care-giver could be an expert or non-expert. Neuromarker information could be displayed along with other patient data, such as, for example, heart rate, blood pressure, body temperature, or other pertinent data.

In some embodiments, a neuromarker display system could include a receiver configured to receive a signal associated with neuronal activity of a mammalian brain. The system could further include a processor configured to process the signal using a non-linear algorithm to extract a signal feature. Further, a neuromarker may be determined using an association between the signal feature and a library of features, as described above in detail. The system could also include a storage system configured to store the library of features, wherein the library could include a plurality of signal features correlated with a plurality of brain states. A display system could be configured to display a representation of the neuromarker.

In some embodiments, neuromarker 50 can be used in conjunction with remote telemetry. For example, a solider injured during battle may have their neuromarker information sent from the battle ground via satellite to a Doctor in a remote location.

In another embodiment, neuromarker 50 can be determined by comparing a feature set extracted from a patient's brain activity with a stored feature set. Based on a known association between a disease state and a feature set, a neuromarker can be determined. Such a neuromarker may also require assessment to confirm that the neuromarker accurately correlates with a disease state. Assessment can include providing a human brain with a known disease state, and receiving a signal associated with neuronal activity of the human brain. The method can also include processing the signal using a non-linear algorithm to extract a signal feature, correlating the signal feature with the known disease state, and identifying a neuromarker based on the signal feature.

Neuromarker data may also require validation to ensure a feature or a feature set is suitably associated with a disease state. For example, validation may include providing a biological indicator associated with a known disease state. The validation process may further require correlating neuromarker 50 with a biological indicator, or other progressive indicator associated with a disease state. In some embodiments, the biological indicator could include imaging data, chemical data, molecular data, or patient test data. For example, imaging data could PET, function MRI, or x-ray data, chemical data could include drug, or ADME pharmacokinetics information, and molecular data could include a binding, RT-PCR, blot, or assay data. In some instances, validation may further include correlating a known disease or brain state with age, gender, physical condition, mental state, or disease progression. In some instances, one or more indicators may be associated with one or more neuromarkers. Also, neuromarker 50 may correlate with other markers, such as, for example, blood pressure, ECG data, heart rate, and so forth. Other validation steps may also be required.

Embodiments consistent with the present invention, using advanced signal processing algorithms and stored data of brain activity of multiple patients having different disease states, may provide a rapid and accurate neuromarker. Moreover, the advanced signal processing algorithms may be executed by a processor capable of integration in a portable handheld device. The portable handheld device used with a reduced electrode set can allow for a rapid, on-site solution for disease monitoring. Such a portable device is described by U.S. Patent No. 6,866,639, and U.S. Patent No. 6,974,421. Also, an appropriate course of treatment, and the efficacy of such treatment, can be readily determined using the system and method of the present disclosure.

Other embodiments will be apparent to those skilled in the art from consideration of the specification and practice of the devices and methods disclosed herein. It is intended that the specification and examples be considered as exemplary only, with a true scope being indicated by the following claims.

## Claims

1. A system (10) configured to display a neuromarker (50), comprising:

a receiver configured to receive a signal (30) associated with neuronal activity of a patient's brain;

a processor configured to process the signal (30) using a non-linear algorithm to extract a signal feature (40) and determine a neuromarker (50) based on an association between the signal feature (40) and a library of features;

a storage system configured to store the library of features, wherein the library includes a plurality of signal features correlated with a plurality of disease states;

a display system configured to display a representation of the neuromarker (50); **characterized in that** the processor is further configured to classify the neuromarker (50) based on a Linear Discriminant Analysis "LDA" employing a two category classifier to assign a discriminant score to the neuromarker (50), the discriminant score indicating the likelihood of a disease state being associated with the neuromarker (50); and the discriminant score $S_A$ associated with a state $A$ is calculated using a Fisher LDA formula

$$S_A = \frac{100 \cdot G(1)}{(G(1) + G(2))}$$

$$G(1) = e^{(Z \cdot W_A + C_A)}, G(2) = e^{(Z \cdot W_B + C_B)}$$

where $Z$ denotes a set of age-regressed and z-transformed features computed for the patient; $W_A$ and $W_B$ denote two weight vectors derived from a reference database, and $C_A$ and $C_B$ are bias constants.

2. The system of claim 1, wherein the signal includes an electro-encephalography signal.

3. The system of any one of the claims 1 or 2, where the signal includes a high frequency band.

4. The system of any one of the claims 1 to 3, wherein the non-linear algorithm is based on at least one of wavelet analysis, diffusion geometric analysis, fractal analysis, and spectral analysis.

5. The system of any one of the claims 1 to 4, wherein the processor is further configured to pre-process the signal, wherein pre-processing includes at least one of denoising, filtering, windowing, sampling, and digitizing.

6. The system of any one of the claims 1 to 5, wherein the library of features is at least partially determined using a genetic algorithm.

7. The system of any one of the claims 1 to 6, wherein the system is further configured to apply a treatment to the patient.

**Patentansprüche**

1. System (10), das konfiguriert ist, um einen Neuromarker (50) anzuzeigen, das Folgendes umfasst:

   einen Empfänger, der konfiguriert ist, um ein Signal (30) zu empfangen, das mit einer neuronalen Aktivität des Gehirns eines Patienten verknüpft ist;
   einen Prozessor, der konfiguriert ist, um das Signal (30) unter Verwendung eines nichtlinearen Algorithmus zu verarbeiten, um ein Signalmerkmal (40) zu extrahieren und, um einen Neuromarker (50) zu bestimmen, basierend auf einer Verknüpfung zwischen dem Signalmerkmal (40) und einer Bibliothek von Merkmalen;
   ein Speichersystem, das konfiguriert ist, um die Bibliothek von Merkmalen zu speichern, wobei die Bibliothek mehrere Signalmerkmale beinhaltet, die mit mehreren Erkrankungszuständen korreliert sind;
   ein Anzeigesystem, das konfiguriert ist, um eine Darstellung des Neuromarkers (50) anzuzeigen;
   **dadurch gekennzeichnet, dass** der Prozessor ferner konfiguriert ist, um den Neuromarker (50) basierend auf einer linearen Diskriminanzanalyse (LDA) zu klassifizieren, wobei ein Klassifikator mit zwei Kategorien genutzt wird, um dem Neuromarker (50) einen Diskriminanzwert zuzuweisen, wobei der Diskriminanzwert die Wahrscheinlichkeit eines Erkrankungszustands angibt, der mit dem Neuromarker (50) verknüpft ist; und
   wobei der Diskriminanzwert SA, der mit einem Zustand A verknüpft ist, unter Verwendung einer Fisher-LDA-Formel berechnet wird

$$S_A = \frac{100.\, G(1)}{(G(1) + G(2))}$$

$$G(1) = e^{(Z.W_A + C_A)}, G(2) = e^{(Z.W_B + C_B)}$$

   wobei Z einen Satz von altersregressiven und z-transformierten Merkmalen bezeichnet, die für den Patienten berechnet werden; $W_A$ und $W_B$ zwei Gewichtsvektoren bezeichnen, die von einer Referenzdatenbank abstammen, und $C_A$ und $C_B$ Vorspannungskonstanten sind.

2. System nach Anspruch 1, wobei das Signal ein Elektroenzephalografie-Signal beinhaltet.

3. System nach einem der Ansprüche 1 oder 2, wobei das Signal ein Hochfrequenzband beinhaltet.

4. System nach einem der Ansprüche 1 bis 3, wobei der nichtlineare Algorithmus auf einer Wavelet-Analyse, einer diffusionsgeometrischen Analyse, einer Fraktalanalyse und/oder einer Spektralanalyse basiert.

5. System nach einem der Ansprüche 1 bis 4, wobei der Prozessor ferner konfiguriert ist, um das Signal vorzuverarbeiten, wobei das Vorverarbeiten Entrauschen, Filtern, Fenstern, Abtasten und/oder Digitalisieren beinhaltet.

6. System nach einem der Ansprüche 1 bis 5, wobei die Bibliothek von Merkmalen wenigstens teilweise durch Verwenden eines genetischen Algorithmus bestimmt wird.

7. System nach einem der Ansprüche 1 bis 6, wobei das System ferner konfiguriert ist, um eine Behandlung an dem Patienten anzuwenden.


**Revendications**

1. Système (10) configuré pour afficher un neuromarqueur (50), comprenant :

   un récepteur configuré pour recevoir un signal (30) associé à l'activité neuronale du cerveau d'un patient ;
   un processeur configuré pour traiter le signal (30) au moyen d'un algorithme non linéaire pour extraire une caractéristique de signal (40) et déterminer un neuromarqueur (50) sur la base d'une association entre la caractéristique de signal (40) et une bibliothèque de caractéristiques ;
   un système de stockage configuré pour stocker la bibliothèque de caractéristiques, dans lequel la bibliothèque comporte une pluralité de caractéristiques de signal corrélées avec une pluralité d'états pathologiques ;
   un système d'affichage configuré pour afficher une représentation du neuromarqueur (50) ;

**caractérisé en ce que** le processeur est en outre configuré pour classer le neuromarqueur (50) sur la base d'une analyse discriminante linéaire "LDA" utilisant un classificateur à deux catégories pour attribuer un score discriminant au neuromarqueur (50), le score discriminant indiquant la probabilité d'un état pathologique étant associé au neuromarqueur (50) ; et
le score discriminant $S_A$ associé à un état $A$ est calculé au moyen d'une formule Fisher LDA

$$S_A = \frac{100.\, G\,(1)}{(G(1) +\ G(2))}$$

$$G(1) = e^{(Z.W_A + C_A)}, G(2) = e^{(Z.W_B + C_B)}$$

où $Z$ désigne un ensemble de caractéristiques régressées par l'âge et transformées en z calculées pour le patient ; $W_A$ et $W_B$ désignent deux vecteurs de poids dérivés d'une base de données de référence, et $C_A$ et $C_B$ sont des constantes de biais.

2. Système selon la revendication 1, dans lequel le signal comporte un signal d'électroencéphalographie.

3. Système selon l'une quelconque des revendications 1 ou 2, dans lequel le signal comporte une bande haute fréquence.

4. Système selon l'une quelconque des revendications 1 à 3, dans lequel l'algorithme non linéaire est basé sur au moins une analyse d'ondelettes et/ou une analyse géométrique de diffusion et/ou une analyse fractale et/ou une analyse spectrale.

5. Système selon l'une quelconque des revendications 1 à 4, dans lequel le processeur est en outre configuré pour prétraiter le signal, dans lequel le prétraitement comporte au moins le débruitage et/ou le filtrage et/ou le fenêtrage et/ou l'échantillonnage et/ou la numérisation.

6. Système selon l'une quelconque des revendications 1 à 5, dans lequel la bibliothèque de caractéristiques est au moins partiellement déterminée au moyen d'un algorithme génétique.

7. Système selon l'une quelconque des revendications 1 à 6, dans lequel le système est en outre configuré pour appliquer un traitement au patient.

**FIG. 1**

**EP 2 498 676 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009045449 A
- US 059014
- US 106699
- US 195001
- US 041106
- US 106657
- US 7054453 B
- US 7054454 B
- US 7302064 B
- US 7333619 B
- WO 2006034024 A
- US 105439
- US 12106657 B
- US 20070032737 A1
- US 2007032737
- US 541272
- US 6866639 B
- US 6974421 B

**Non-patent literature cited in the description**

- **ANASTASSIOU.** Computational Analysis of the Synergy Among Multiple Interacting Genes. *Molecular Systems Biology,* 2007, vol. 3, 83
- **ERGÜN et al.** A Network Biology Approach to Prostate Cancer. *Molecular Systems Biology,* 2007, vol. 3, 82
- **DAVATZIKOS et al.** Classifying Spatial Patterns of Brain Activity With Machine Learning Methods: Application to Lie Detection. *Neurolmage,* 2005, vol. 28, 663-668
- **TOGNOLI et al.** The Phi Complex as a Neuromarker of Human Social Coordination. *PNAS,* 2007, vol. 104 (19), 8190-8195